Europäisches Patentamt

(19) European Patent Office

Office européen des brevets

(11) Numéro de publication: **0 093 630**
**B1**

(12) **FASCICULE DE BREVET EUROPEEN**

(45) Date de publication du fascicule du brevet:
25.09.85

(51) Int. Cl.⁴: **A 61 D 7/02**

(21) Numéro de dépôt: 83400691.8

(22) Date de dépôt: 01.04.83

(54) Chemise de protection contre les contaminations internes pour pistolet gynécologique, notamment pour bovins.

(30) Priorité: 29.04.82 FR 8207420

(43) Date de publication de la demande:
09.11.83 Bulletin 83/45

(45) Mention de la délivrance du brevet:
25.09.85 Bulletin 85/39

(84) Etats contractants désignés:
AT BE CH DE GB IT LI LU NL SE

(56) Documents cités:
DE - A - 2 435 790
FR - A - 1 344 427
FR - A - 1 434 411
FR - A - 2 386 312
FR - A - 2 468 522

(73) Titulaire: Cassou, Robert, Rue Clémenceau,
F-61300 L'Aigle (FR)
Titulaire: Cassou, Maurice, Rue Clémenceau,
F-61300 L'Aigle (FR)
Titulaire: Cassou, Bertrand, Saint Symphorien des
Bruyeres, F-61300 L'Aigle (FR)

(72) Inventeur: Cassou, Robert, Rue Clémenceau,
F-61300 l'Aigle (FR)

(74) Mandataire: Rodhain, Claude, Cabinet Claude
RODHAIN 30, rue La Boétie, F-75008 Paris (FR)

# Description

La présente invention concerne une chemise de protection pour pistolets gynécologiques pour animaux, tels que les pistolets d'insémination artificielle ou de transfert ou collecte d'embryons pour le bétail ou les volailles, lesdits pistolets étant du type comprenant un corps tubulaire, une capacité de stockage de produit à injecter ou aspirer et des moyens d'éjection ou d'aspiration par l'extrémité avant du corps. Ce corps peut être complété par une gaine semi-rigide qui enveloppe le corps tubulaire et est fixée de manière amovible autour d'une extrémité de préhension de celui-ci. Cette gaine a pour but d'éviter tout contact du corps de pistolet lui-même avec l'animal et donc toute contamination postérieure d'un autre animal lors d'une nouvelle utilisation étant donné que l'on jette et remplace cette gaine après chaque opération, de sorte que le corps de pistolet peut demeurer stérile pendant un nombre illimité d'opérations.

On connaît déjà, par exemple par les documents de brevet français publiés sous les Nos 1224918, 1467943, 1525336 ou 2358136, des pistolets d'insémination de ce type, notamment pour bovins, dans lesquels le corps est destiné à recevoir, à son extrémité avant, une réserve de produit d'insémination en forme de paillette tandis que les moyens d'éjection sont constitués par une tige-poussoir qui est montée coulissante dans ce corps du côté de son extrémité arrière. Il est en général prévu une gaine de protection contre les contaminations externes qui s'étend pratiquement sur toute la longueur du corps et offre à son extrémité avant une partie convergente, comportant éventuellement un manchon intérieur, contre l'intérieur de laquelle s'appuie l'extrémité avant de la paillette, cette extrémité convergente présentant bien entendu une ouverture d'éjection en regard de cette extrémité.

On connaît aussi, par exemple par les documents de brevets français publiés sous les Nos 2477005 et 2477006, des pistolets de transfert, ou de simple collecte d'embryons, également pour bovins, qui sont du même type et dans lesquels la capacité de stockage des embryons est réalisée soit sous la forme d'une paillette, comme dans le cas de l'insémination, soit sous la forme d'une chambre intérieure ménagée à l'avant du corps, soit encore (dans le cas de la simple collecte) sous la forme d'une capacité séparée reliée à l'arrière du corps, tandis que, en cas de transfert, les moyens d'éjection sont encore constitués par une tige-poussoir montée coulissante à l'arrière du corps. Dans les différentes variantes de transfert, il peut toujours être prévu une gaine de protection contre les contaminations extérieures et qui s'étend soit sur toute la longueur du corps pour servir d'appui à l'extrémité avant de celui-ci dans le cas d'une paillette, soit le long d'une partie principale extérieure du corps, qui est réalisée télescopique, dans le cas d'une chambre interne de stockage.

Il se trouve toutefois que de telles gaines de protection contre les contaminations externes ne luttent efficacement que contre la contamination due au passage d'un animal au suivant. Or, il est connu que vivent dans le vagin des bovins des mycoplasmes qui sont des microbactéries qui, si elles pénètrent dans l'utérus, réduisent notablement le taux de fécondation, surtout dans certaines régions géographiques où les mycoses se développent plus aisément. L'action néfaste de ces mycoplasmes dans l'utérus peut se produire malgré la présence d'anticorps spécialement secrétés par l'utérus pour lutter contre eux. La gaine de protection risque donc de transférer de tels microplasmes du vagin jusque dans l'utérus.

Le même inconvénient pourrait également se présenter dans le cas de pistolets sans gaine de protection, par exemple du type jetable (et à extrémité non rigide afin de ne pas blesser l'animal), auquel cas ce serait l'extrémité du corps lui-même qui risquerait de transférer les mycoplasmes du vagin vers l'utérus.

C'est pourquoi l'invention a pour but de supprimer cet inconvénient en permettant de disposer d'un pistolet du type précité dont l'extrémité, éventuellement munie d'une gaine de protection contre les contaminations externes (d'un animal à l'autre), ne risque pas non plus de transférer, du vagin vers l'utérus de l'animal lui-même, les micro-organismes, pathogènes ou non, susceptibles de se développer ou non, qui siègent sur les parois de la vulve et du vagin et pourraient, s'ils étaient introduits dans l'utérus, provoquer des troubles néfastes, notamment de la fécondation. Ce résultat doit pouvoir être obtenu aussi bien dans la pratique de l'insémination artificielle que dans la transplantation des embryons (inovulation) ou encore du prélèvement des germes qui se développent dans la glaire du vagin. Une autre condition impérative devant être respectée par la solution apportée à ce problème par l'invention réside dans le fait que cette protection ne doit exiger, pour son application, que l'utilisation de la seule et même main qui tient le pistolet et le manœuvre étant donné que l'autre main et le bras se trouvent déjà introduits dans le rectum de l'animal pour palper la paroi qui avoisine le col de l'utérus et permettre une bonne localisation de celui-ci et une pénétration précise et sans blessure, de l'instrument à travers ce col.

A cet effet, l'invention a pour objet une chemise jetable de protection contre les contaminations microbactériennes internes entre le vagin et l'utérus d'un même animal pour pistolet du type précité, ladite chemise étant constituée par un élément tubulaire à paroi mince en matière souple qui, en opération, enveloppe l'ensemble du pistolet, une extrémité dudit élément étant obturée de manière étanche, mais déchirable, la longueur dudit élément correspondant approximativement à celle de l'ensemble du pistolet, deux fentes à peu près parallèles étant ménagées à proximité de l'extrémité opposée à ladite extrémité obturée.

Grâce à cette disposition, avant usage, l'utilisateur enfile la chemise sur le corps du pistolet (ou sur sa gaine, s'il en est prévu une) jusqu'à ce que l'extrémité de ce corps (ou de cette gaine) vienne buter contre l'extrémité obturée de cette chemise, à la suite de quoi, il introduit l'ensemble dans le vagin de l'animal, à l'aide d'une seule main, jusqu'à ce que l'extrémité obturée de la chemise, qui retient le corps de pistolet, ait commencé à pénétrer dans le col de l'utérus ou soit sur le point de le faire. L'utilisateur passe alors l'index de la main qui tient le pistolet dans les deux fentes prévues dans la chemise et tire alors sur la partie de la chemise qui sépare ces deux fentes et n'entoure pas le pistolet, de manière à former, du fait de la souplesse et de l'élasticité de la matière constitutive de la chemise, un demi-anneau ou anse, par lequel il applique sur la chemise une traction en prenant appui à l'aide du pouce de la même main sur le corps du pistolet, de sorte que l'extrémité de ce pistolet (ou de sa gaine) perfore l'extrémité obturée de la chemise contre laquelle elle venait en butée. A la suite de cette perforation l'utilisateur peut introduire, toujours à l'aide de la même main, le pistolet à l'intérieur de l'utérus, et ceci jusqu'à ce que son extrémité atteigne la position optimale voulue pour l'opération à effectuer. Ainsi, pendant toute la traversée de la vulve et du vagin, le corps de pistolet (ou sa gaine) a été entièrement maintenu hors de contact avec la paroi et n'a donc pu être contaminé par les micro-organismes, alors que la chemise, une fois perforée, demeure en deçà du col de l'utérus. La protection recherchée se trouve donc obtenue et sa mise en œuvre ne nécessite que l'utilisation de la seule et même main qui tient normalement le pistolet, tandis qu'en outre la pénétration dans l'utérus peut être aussi profonde que nécessaire puisque, du fait de sa souplesse, la chemise peut se plisser autour du pistolet au fur et à mesure de son mouvement sans, en aucune manière, limiter la course de ce dernier.

Dans un mode de réalisation particulier de l'invention, il est prévu que l'élément tubulaire soit venu d'extrusion et soit aplati de manière à former deux couches superposées, l'extrémité obturée de cette chemise étant constituée par une solidarisation desdites couches transversalement à l'axe de l'élément tubulaire, de sorte que la fabrication de ces chemises est très aisée s'obtenant à l'aide d'un procédé simple d'extrusion à laquelle sont adjoints des organes de solidarisation, de préférence de thermosoudure, et de réalisation de fentes. On pourrait également envisager de réaliser ce tube par la superposition et la soudure en continu de deux bandes de matière identique ou différente, associant par exemple, dans le cas d'une bande de matière plastique et d'une bande de papier souple, l'élasticité de l'une et l'inextensibilité de l'autre, cette association garantissant à 100% la perforation de la chemise.

De manière particulièrement avantageuse, chaque chemise peut faire partie d'un tube extrudé continu et aplati constitué par la juxtaposition axiale d'une série de telles chemises, tandis que ce tube est enroulé axialement sous la forme d'un rouleau qui est contenu dans un chargeur de forme générale cylindrique et présentant une fente par laquelle sort ledit tube, de sorte que l'utilisateur dispose d'un ensemble de chemises sous un très faible encombrement et facile à distribuer, le tube se déroulant sous une simple traction, tandis que la séparation des chemises successives se fait par simple déchirement ou procédé de découpe.

D'autres caractéristiques et avantages de l'invention ressortiront de la description qui va suivre, à titre d'exemples non limitatifs et en regard des dessins annexés sur lesquels:

la fig. 1 représente une vue, en coupe et avec arrachement, d'un pistolet pouvant être équipé d'une chemise conforme à l'invention;

la fig. 2 représente une vue analogue du même pistolet représenté en extérieur et revêtu d'une chemise suivant un premier mode de réalisation conforme à l'invention;

la fig. 3 illustre l'utilisation de ce pistolet muni d'une chemise de protection conforme à l'invention;

la fig. 4 représente l'ensemble d'une série de telles chemises réalisées sous la forme d'un tube aplati continu présenté sous forme d'un rouleau contenu dans un chargeur;

les fig. 5 et 6 représentent deux réalisations différentes de chemises réalisées sous la forme d'un tube aplati en étant juxtaposées dans le même sens;

les fig. 7, 8 et 9 représentent trois autres modes de réalisation dans lesquels les chemises sont réalisées sous forme de tubes aplatis avec juxtaposition des chemises deux par deux tête-bêche;

la fig. 10 illustre un conditionnement en chaîne de rouleaux du type de la fig. 4;

la fig. 11 illustre la présentation d'une pile de chemises sous forme brochée.

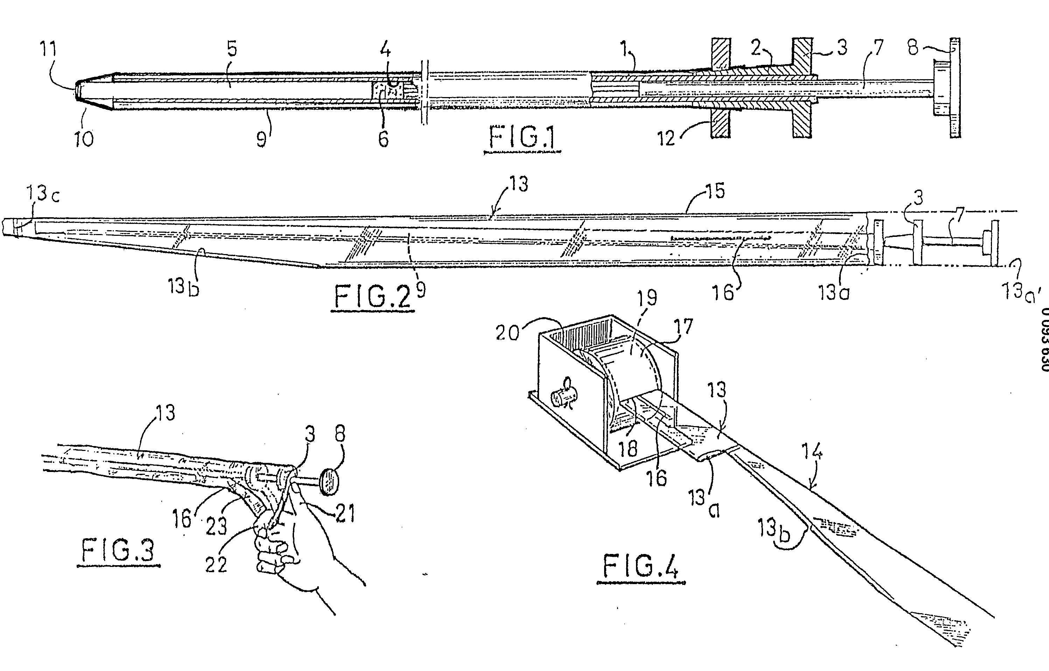

Le pistolet représenté sur la fig. 1 est un pistolet d'insémination artificielle, notamment pour bovins, qui comprend un corps tubulaire 1, muni à une extrémité d'une tête 2 de fixation de gaine qui se termine, elle-même, par une collerette annulaire 3 d'appui des doigts, tandis qu'à son autre extrémité, il offre un épaulement intérieur 4 dans lequel vient s'emboîter une extrémité d'une réserve de semence ou paillette 5 munie d'un bouchon-piston 6. Il comprend, en outre, une tige-poussoir 7 montée coulissante dans le corps 1 de manière à pouvoir venir, à une extrémité, repousser le bouchon-piston 6 et donc éjecter la semence contenue dans la paillette 5, tandis qu'à son autre extrémité, elle offre une collerette 8 d'appui des doigts. Le corps 1 et cette tige 7 sont, par exemple, réalisés en acier inoxydable. Le pistolet est enfin complété par une gaine cylindrique de protection 9 qui recouvre l'ensemble du corps 1 de la paillette 5, cette dernière venant, par son extrémité avant, s'appuyer sur la face intérieure d'une partie convergente 10 de cette gaine qui présente un orifice d'éjection 11, tandis qu'à son autre extrémité, la gaine est élastiquement serrée sur une partie tronconique de la tête 2, grâce à une bague de serrage 12.

Une telle gaine 9, jetable, est utilisée pour chaque opération d'insémination et est enfilée sur le pistolet avant son introduction dans les voies génitales de l'animal, son extrémité avant d'éjection 10 pouvant s'arrêter avant le col de l'utérus, ou même le dépasser. Cette gaine est jetée une fois l'appareil retiré des voies génitales de l'animal, de sorte que le pistolet proprement dit 1 n'est pas souillé et peut être utilisé sans inconvénient sur un autre animal, recouvert d'une nouvelle gaine.

Comme le montre la fig. 2, un tel pistolet, muni de sa gaine 9, se trouve enveloppé dans une chemise de protection 13 qui est destinée à éviter que la gaine 9 ne recueille au passage dans la vulve et le vagin des micro-organismes qu'elle transférerait dans l'utérus. Cette chemise 13 est constituée par une section de tube extrudé en une matière très souple et très fine telle que, par exemple, du polyéthylène, de la cellophane, du papier biodégradable, ou la combinaison de deux de ces éléments, d'une épaisseur, par exemple de 25 microns. Cette chemise présente une extrémité 13a qui est pratiquement ouverte sur toute sa surface et par laquelle le pistolet a pu être enfilé à l'intérieur de la chemise, tandis que son extrémité opposée 13b est obturée de manière étanche. L'extrémité 10 de la gaine 9 du pistolet vient buter contre la partie la plus antérieure 13c de cette extrémité 13b, la longueur de la chemise, comprise entre cette partie 13c et l'extrémité 13a, étant telle qu'elle enveloppe entièrement le pistolet jusqu'à hauteur de la tête 2 de fixation de la gaine, et même au-delà de la collerette 8 si nécessaire (extrémité 13a' en trait mixte).

Comme le montre plus précisément la fig. 4, une telle chemise 13 fait partie d'un tube extrudé continu et aplati 14 constitué par la juxtaposition axiale d'une série de telles chemises 13. Ce tube, et par conséquent chacune des chemises 13, est aplati, de manière à former deux couches superposées reposant l'une sur l'autre. L'extrémité 13b de chaque chemise est obturée de manière étanche par solidarisation des deux couches entre elles, par thermosoudure, soudure par impulsion, ultrasons ou tout autre procédé, le long d'une ligne droite très fortement inclinée, par rapport à la direction transversale de la chemise, par exemple de 75°. Cette ligne de soudure 13b converge avec l'un des deux bords 15 de la chemise aplatie, mais ne s'étend pas jusqu'à leur intersection, se trouvant interrompue par une autre ligne de soudure relativement courte et orientée transversalement à la chemise, qui constitue la partie la plus antérieure 13c de cette extrémité avant obturée 13b.

Comme le montre également la fig. 4, les chemises 13 successives qui constituent le tube extrudé et aplati 14 sont juxtaposées en étant toutes disposées dans le même sens, ce qui signifie que la ligne de solidarisation par thermosoudure 13b, 13c d'une chemise se trouve être directement adjacente à l'extrémité ouverte 13a de la chemise suivante, une chute de forme triangulaire ayant été supprimée à la fabrication entre la ligne fortement inclinée 13b et l'extrémité ouverte 13a adjacente.

Comme le montre encore la fig. 4, chaque chemise 13 présente, à proximité de son extrémité ouverte 13a, deux fentes 16 qui sont parallèles à l'axe principal de la chemise tubulaire et sont même exactement superposées ou coïncidentes avec l'axe de la chemise lorsque les deux couches de la chemise reposent à plat l'une sur l'autre.

La section transversale de la chemise 13 est relativement importante et peut correspondre à plusieurs fois celle du corps de pistolet 1 et de la gaine de protection 9 qui le recouvre, de manière à envelopper ces derniers de manière relativement libre, même au niveau de l'extrémité avant où la pointe 10 de la gaine est entourée avec du jeu par l'extrémité convergente qui forme la ligne 13b avec le bord 15 de la chemise. Ce jeu évite que la pointe conique 10 de la gaine ne se trouve «étranglée» pendant son mouvement de pénétration, avant perforation de l'extrémité de la chemise, mouvement provoquant à la fois une élongation axiale et un rétreint radial qui serait responsable d'un tel étranglement; à l'extrême, ce dernier entraînerait l'arrachement d'un cône complet de matière qui demeurerait sur l'extrémité de la gaine et pénétrerait ainsi par le col de l'utérus, avec les risques de corps étranger et de contamination correspondants, que l'on cherche justement à éviter.

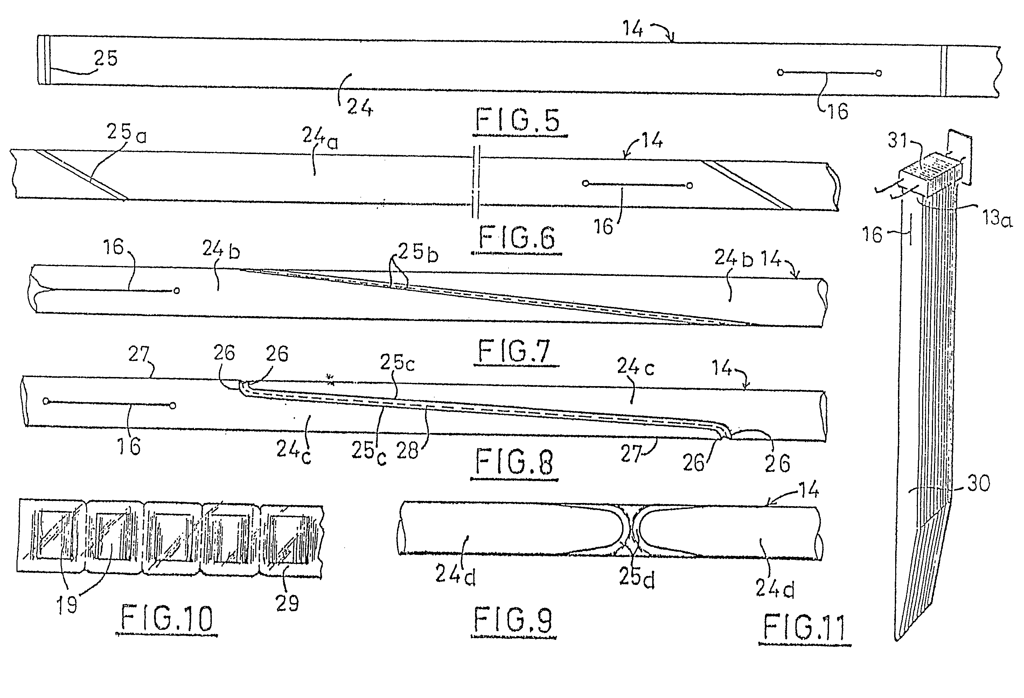

Dans le cas de réalisation particulière illustrée par la fig. 2, la largeur de la chemise aplatie pardessus le pistolet est de l'ordre de trois fois le diamètre de ce dernier. Par ailleurs, les fentes 16 sont disposées approximativement au milieu de la largeur de la chemise aplatie, comme le montrent les fig. 2 et 5. On constate donc bien que cette double disposition permet au corps de pistolet 1 d'être placé à l'intérieur de la chemise 13, sur l'un des côtés de cette dernière, de manière à être situé entièrement sur un seul et même côté de l'ensemble des deux fentes 16, ce qui permettra, comme cela sera décrit par la suite, l'introduction aisée de l'index dans l'une et l'autre de ces fentes.

Comme le montre également la fig. 5, le tube 14 qui est constitué d'un très grand nombre de chemises 13, se succédant, par exemple de l'ordre de plusieurs dizaines, se trouve enroulé axialement sous la forme d'un rouleau de chemise appelé charge 19, placé dans un chargeur de forme générale cylindrique 17, réalisé par exemple en matière plastique ou en acier inoxydable, et qui présente une fente 18 parallèle à son axe et hors de laquelle fait saillie l'extrémité du tube enroulé 14. Ce chargeur cylindrique 17 peut s'ouvrir de façon à recevoir la charge et être placé dans un boîtier distributeur, présentoir ou trousse de travail 20, que l'utilisateur peut placer ou fixer à volonté, où bon lui semble, dans un local ou sur une caisse de travail par exemple.

L'utilisation des chemises de protection ainsi décrites est la suivante:

L'extrémité 13a d'un rouleau de chemises dépassant hors de la fente 18 du chargeur, l'utilisateur saisit cette dernière et procède à une traction jusqu'à ce que l'ensemble d'une chemise 13 en soit issue, à la suite de quoi, il déchire ou coupe cette chemise complète au-delà de la soudure extrême 13c, complétant ainsi, de ce fait, l'ouverture 13a de la chemise suivante. Il introduit alors l'ensemble du pistolet 1 et de la paillette 5 déjà recouverts préalablement par la gaine 9, à l'intérieur de cette chemise 13 et à l'aide de l'ouverture extrême d'entrée 13a, ceci jusqu'à ce que l'extrémité 10 de la gaine vienne en butée contre la ligne extrême de soudure 13c. Dans cette position, les deux collerettes 3 et 8 du pistolet ainsi que la bague 12 peuvent demeurer à l'extérieur de la chemise 13 au-delà de son ouverture 13a, tandis que le pistolet est placé sur l'un des côtés de la chemise, de manière à ne pas s'interposer entre les deux fentes 16.

L'utilisateur introduit alors l'ensemble ainsi constitué par le pistolet et la chemise 13 dans les voies génitales de l'animal, en traversant successivement la vulve et le vagin, et ceci jusqu'à ce que l'extrémité avant 13c de la chemise soit sensiblement en regard du col de l'utérus, ce positionnement s'effectuant à la manière connue grâce à la mise en place de l'autre main dans le rectum de l'animal.

Se servant alors de la main à l'aide de laquelle il a mis en place le pistolet revêtu de sa chemise, l'utilisateur introduit l'index à travers les deux fentes 16 de la chemise, et, prenant appui à l'aide du pouce 21 sur la collerette 3 du pistolet, il procède, à l'aide de l'index 22, à une traction sur le demi-anneau ou anse 23 que forme alors la partie de la chemise située sur le côté des fentes 16, en raison de la déformation plastique aisée du matériau utilisé. Cette traction se propage tout le le long de la chemise 13 et agit ainsi sur l'extrémité soudée 13c de cette dernière, de sorte que l'extrémité convergente 10 de la gaine du pistolet perfore cette dernère et la traverse.

Cette extrémité 13c percée se trouvant ainsi en retrait de quelques millimètres par rapport à l'orifice d'éjection 11 de la gaine, l'utilisateur peut alors faire pénétrer l'ensemble du pistolet revêtu de la gaine 9 vers le col de l'utérus, et ceci jusqu'à ce que son extrémité se trouve placée dans la position optimale souhaitée pour l'opération d'insémination. Pendant cette pénétration, la chemise 13 se plisse le long de la gaine 9, puisque se trouvant retenue en arrière du col de l'utérus. Grâce à cette disposition, et plus particulièrement cette possibilité de plissage qui ne présente aucune limitation, la pénétration à l'intérieur de l'utérus peut être aussi profonde que nécessaire.

Une fois l'insémination effectuée, l'utilisateur extrait l'ensemble et jette successivement la chemise 13 et la gaine 9, le pistolet 1 se trouvant ainsi maintenu non souillé et prêt pour une nouvelle opération.

Dans la variante de réalisation illustrée par la fig. 5, l'extrémité obturée de chaque chemise 24 faisant partie d'un tube extrudé 14 se trouve constituée par une ligne de soudure 25 transversale, c'est-à-dire perpendiculaire à la longueur de la chemise. Les fentes 16 présentent toujours la même disposition et, lors du prélèvement à partir d'un chargeur 17, l'extrémité ouverte 13a de la chemise a déjà été réalisée lors de la séparation de la chemise précédente, au-delà de la ligne de soudure 25 de celle-ci.

Dans la variante de réalisation illustrée par la fig. 6, la ligne de soudure 25a qui constitue l'extrémité obturée de la chemise 24a se trouve inclinée à approximativement 45° par rapport à l'axe de la chemise.

Dans l'une et l'autre des deux réalisations précédentes, les chemises successives 24 ou 24a appartenant à un même tube extrudé 14 sont toutes disposées dans le même sens les unes derrière les autres; par contre, dans la réalisation illustrée par la fig. 7, les chemises 14b du tube 14 sont, deux par deux, regroupées tête-bêche, de manière que soient juxtaposées les deux lignes de soudure 25b constituant les extrémités obturées de ces chemises. Les deux lignes de soudure 25b juxtaposées sont disposées suivant des lignes droites très fortement inclinées par rapport à la transversale, par exemple à environ 75°. Dans ce cas, l'arrachement des chemises successives se fait alternativement, une fois le long des lignes de soudure 25b et l'autre fois suivant une ligne transversale non représentée sur la figure, et suivant laquelle sont juxtaposées les extrémités ouvertes 13a de deux chemises.

Dans la variante de réalisation de la fig. 8, les chemises 24c sont également regroupées deux par deux tête-bêche, mais les lignes de soudure correspondantes 25c sont, à chacune de leurs deux extrémités, interrompues par un arrondi 26 ou une courte soudure transversale, se raccordant au bord 27 de la chemise maintenue à plat. En outre, les deux lignes de soudure 25c sont séparées par une ligne d'affaiblissement 28 réalisée à travers l'épaisseur des deux couches constitutives à la chemise, par exemple par une succession de perforations.

Dans la variante de la fig. 9, les chemises 24d sont également regroupées deux par deux tête-bêche et les lignes de soudure 25d présentent des formes semi-circulaires s'étendant sur toute la largeur des chemises et tangentes entre elles.

Le rouleau de chemises ou charge 19 est, une fois terminé, interchangeable et ces charges 19 peuvent être conditionnées ou présentées sous la forme d'une chaîne continue d'alvéoles successifs 29 qui se présentent par groupes, par exemple d'une ou plusieurs dizaines, comme le montre la fig. 10.

Dans une variante de conditionnement, les chemises 30 peuvent être présentées sous la forme unitaire, c'est-à-dire non maintenues sous forme d'un tube continu, ces différentes chemises 30 constituant une pile de chemises présentant la même orientation et dont les extrémités ouvertes 13a sont maintenues par un brochage 31, ainsi que représenté par exemple sur la fig. 11.

**Revendications**

1. Chemise jetable de protection contre les contaminations microbactériennes internes entre le vagin et l'utérus d'un même animal, pour pistolet gynécologique pour animaux, ledit pistolet étant du type comprenant un corps tubulaire (1), une capacité (5) de stockage du produit à injecter ou aspirer et des moyens (7) d'éjection ou d'aspiration par l'extrémité avant du corps et éventuellement d'une gaine (9) semi-rigide enveloppant ledit corps, ladite chemise étant constituée par un élément tubulaire à paroi mince (13, 24, 24a, 24b, 24c, 24d, 30) qui, en opération, enveloppe l'ensemble du pistolet (1, 7, 9), une extrémité (13c, 25, 25a, 25b, 25c, 25d) dudit élément étant obturée de manière étanche, mais déchirable, la longueur dudit élément correspondant approximativement à celle de l'ensemble du pistolet (1, 7, 9), deux fentes (16) à peu près parallèles étant ménagées à proximité de l'extrémité (13a) opposée à ladite extrémité obturée.

2. Chemise de protection selon la revendication 1, caractérisée en ce que l'élément tubulaire (13, 24, 24a, 24b, 24c, 24d, 30) est venu d'extrusion et est adapté à être aplati de manière à former deux couches superposées, l'extrémité obturée de cette chemise (13c, 25, 25a, 25b, 25c, 25d) étant constituée par une solidarisation desdites couches transversalement à l'axe de l'élément tubulaire.

3. Chemise de protection selon la revendication 1, caractérisée en ce que l'élément tubulaire est adapté aplati et est constitué de deux bandes de matière différente soudées le long de leurs bords.

4. Chemise de protection selon l'une quelconque des revendications 2 et 3, caractérisée en ce que les deux fentes (16) sont formées respectivement dans les deux couches superposées et sont elles-mêmes superposées.

5. Chemise de protection selon l'une quelconque des revendications 1 à 4, caractérisée en ce que les deux fentes (16) sont orientées parallèlement à l'axe de l'élément tubulaire (13, 24, 24a, 24b, 24c, 24d, 30).

6. Chemise de protection selon l'une quelconque des revendications 1 à 5, caractérisée en ce que l'une des deux parties d'une section transversale normale de l'élément tubulaire délimitées par une ligne reliant les points d'intersection entre ladite section et les deux fentes (16), correspond au moins à la section du pistolet (1).

7. Chemise de protection selon l'une quelconque des revendications 2 à 6, lorsque les revendications 5 et 6 dépendent de la revendication 2, caractérisée en ce que les deux couches sont solidarisées suivant une ligne droite (25) perpendiculaire à l'axe de l'élément cylindrique (24).

8. Chemise de protection selon l'une quelconque des revendications 2 et 6, lorsque les revendications 5 à 6 dépendent de la revendication 2, caractérisée en ce que les deux couches sont solidarisées suivant une ligne droite (25a) approximativement aussi inclinée par rapport à l'axe de l'élément cylindrique (24a) que par rapport à la transversale.

9. Chemise de protection selon l'une quelconque des revendications 2 à 6, lorsque les revendications 5 et 6 dépendent de la revendication 2, caractérisée en ce que les deux couches sont solidarisées suivant une ligne droite (25b) très fortement inclinée par rapport à la transversale.

10. Chemise de protection selon la revendication 9, caractérisée en ce que, à chacune de ses deux extrémités, la ligne (25c) est arrondie (26) ou interrompue par une courte ligne transversale.

11. Chemise de protection selon l'une quelconque des revendications 2 à 6, lorsque les revendications 2 et 6 dépendent de la revendication 2, caractérisée en ce que les deux couches sont solidarisées suivant une ligne transversale semi-circulaire (25d).

12. Chemise de protection selon l'une quelconque des revendications 7 à 11, caractérisée en ce qu'elle fait partie d'un tube extrudé continu et aplati (14) constitué par la juxtaposition axiale d'une série de telles chemises (13, 24, 24a, 24b, 24c, 24d).

13. Chemise de protection selon la revendication 12, lorsqu'elle dépend de l'une quelconque des revendications 7 et 8, caractérisée en ce que les chemises (13, 24, 24a) sont juxtaposées en étant toute disposées dans le même sens, la ligne de solidarisation (13b, 13c, 25, 25a) d'une chemise étant directement adjacente à l'extrémité ouverte (13a) de la chemise voisine.

14. Chemise de protection selon la revendication 12 lorsqu'elle dépend de l'une quelconque des revendications 9 à 11, caractérisée en ce que les chemises (24b, 24c, 24d) sont juxtaposées en étant disposées deux par deux tête-bêche, de manière que leurs lignes de solidarisation (25b, 25c, 25d) soient adjacentes.

15. Chemise de protection selon la revendication 14, caractérisée en ce que les lignes de solidarisation (25c) de deux chemises voisines (24c) disposées tête-bêche sont séparées par une ligne d'affaiblissement (28).

16. Chemise de protection selon l'une quelconque des revendications 2 à 15 lorsque les revendications 5 à 15 dépendent de la revendication 2, caractérisée en ce que chaque ligne de solidarisation (13c, 25, 25a, 25b, 25c, 25d) est une ligne soudée.

17. Chemise de protection selon l'une quelconque des revendications 12 à 15 et 16 lorsque celle-ci dépend de l'une quelconque de ces revendications 12 à 15, caractérisée en ce que le tube extrudé continu et aplati (14), dont fait partie cette chemise, est enroulé axialement sous forme d'un rouleau qui est contenu dans un chargeur (17) de forme générale cylindrique et présentant une fente (18) par laquelle sort ledit tube.

18. Chemise de protection selon la revendication 17, caractérisée en ce que le chargeur (17) qui contient le tube dont elle fait partie, est lui-même monté de manière amovible sur un distributeur fixe (20).

19. Chemise de protection selon la revendication 17, caractérisée en ce qu'elle fait partie d'un rouleau de chemise (19) présenté lui-même sous un conditionnement en chaîne (29).

20. Chemise de protection selon l'une quelconque des revendications 7 à 11, caractérisée en ce qu'elle fait partie d'une pile de telles chemises (30) aplaties et superposées en étant disposées dans le même sens, cette pile étant maintenue par un brochage (31) à son extrémité (13a) opposée aux lignes de solidarisation.

## Claims

1. Disposable covering for protection against internal micro-bacterial contamination between the vagina and uterus of the same animal, for a gynaecological applicator for animals, the said applicator being of the type comprising a tubular body (1), storage capacity for the product to be injected or aspirated and ejection and aspiration means (7) via the front end of the body and possibly of a semi-rigid sheath (9) enclosing the said body, the said covering comprising a thin-walled tubular element (13, 24, 24a, 24b, 24c, 24d, 30) of a flexible material which, in operation, encloses the entire applicator (1, 7, 9), one end (13c, 25, 25a, 25b, 25c, 25d) of the said element being sealed in a watertight manner, but able to be torn, the length of the said element corresponding approximately to that of the entire applicator (1, 7, 9), two approximately parallel slots (16) being provided near the end (13a) opposite the said sealed end.

2. Protective covering according to Claim 1, characterised in that the tubular element (13, 24, 24a, 24b, 24c, 24d, 30) is formed by extrusion and is adapted to be flat so as to form two superposed layers, the sealed end of the covering (13c, 25, 25a, 25b, 25c, 25d) comprising the solid interconnection of the said layers transversely to the axis of the tubular element.

3. Protective covering according to Claim 1, characterised in that the tubular element is formed flat and comprises two strips of different material welded along their edges.

4. Protective covering according to either one of Claims 2 and 3, characterised in that the two slots (16) are formed respectively in the two superposed layers and are themselves superposed.

5. Protective covering according to any one of Claims 1 to 4, characterised in that the two slots (16) are arranged parallel to the axis of the tubular element (13, 24, 24a, 24b, 24c, 24d, 30).

6. Protective covering according to any one of Claims 1 to 5, characterised in that one of the two parts of a normal transverse section of the tubular element, defined by a line linking the points of intersection between the said section and the two slots (16), corresponds at least to the section of the applicator (1).

7. Protective covering according to any one of Claims 2 to 6, when Claims 5 and 6 are dependent upon Claim 2, characterised in that the two layers are solidly interconnected along a straight line (25) perpendicular to the axis of the cylindrical element (24).

8. Protective covering according to any one of Claims 2 to 6, when Claims 5 and 6 are dependent upon Claim 2, characterised in that the two layers are solidly interconnected along a straight line (25a) inclined to approximately the same degree relative to the axis of the cylindrical element (24a) as relative to the longitudinal axis and transverse direction.

9. Protective covering according to any one of Claims 2 to 6, when Claims 5 and 6 are dependent upon Claim 2, characterised in that the two layers are solidly interconnected along a straight line (25b) which is very steeply inclined relative to the longitudinal axis and transverse direction.

10. Protective covering according to Claim 9, characterised in that, at each of its two ends, the line (25c) is rounded (26) or interrupted by a short transverse line.

11. Protective covering according to any one of Claims 2 to 6, when Claims 2 and 6 are dependent upon Claim 2, characterised in that the two layers are solidly interconnected along a semi-circular transverse line (25d).

12. Protective covering according to any one of Claims 7 to 11, characterised in that it forms part of a flat, continuous, extruded tube (14) comprising the axial juxtaposition of a series of such coverings (13, 24, 24a, 24b, 24c, 24d, 30).

13. Protective covering according to Claim 12, when it is dependent upon either one of Claims 7 and 8, characterised in that the coverings (13, 24, 24a) are juxtaposed and all being disposed in the same direction, the seam (13b, 13c, 25, 25a) of one covering being directly adjacent to the open end (13a) of the adjacent covering.

14. Protective covering according to Claim 12, when it is dependent upon any one of Claims 9 to 11, characterised in that the coverings (24b, 24c, 24d) are juxtaposed, being disposed two at a time top to bottom in such a way that their seams (25b, 25c, 25d) are adjacent.

15. Protective covering according to Claim 12, characterised in that the seams (25c) of the two adjacent coverings (24c) disposed top to bottom are separated by a line of weakness (28).

16. Protective covering according to any one of Claims 2 to 15, when Claims 5 to 15 are dependent upon Claim 2, characterised in that each seam (13c, 25, 25a, 25b, 25c, 25d) is a welded line.

17. Protective covering according to any one of Claims 12 to 15 and 16, when the latter is dependent upon any one of Claims 12 to 15, characterised in that the flat, continuous, extruded tube (14), of which the said covering is a part, is wound axially in the shape of a roll which is contained in a loader (17) basically cylindrical in shape and having a slot (18) through which the tube emerges.

18. Protective covering according to Claim 17, characterised in that the loader (17), which contains the tube of which it is a part, is mounted in a detachable manner on a fixed distributor.

19. Protective covering according to Claim 17, characterised in that it forms part of a roll of coverings (19) provided in the form of continuous packaging (29).

20. Protective covering according to any of Claims 7 to 11, characterised in that it forms part of a stack of flat and superposed coverings (20) of this type being disposed in the same direction, the stack being held by a tag (31) at its end (13a) opposite the seams.

## Patentansprüche

1. Einwegschutzhülle gegen interne mikrobakterielle Kontamination zwischen der Vagina eines Tieres und seinem Uterus für eine gynäkologische Pistole für Tiere, welche Pistole von der Art mit einem rohrförmigen Körper (1), einem Aufnahmeraum (5) für das zu injizierende oder anzusaugende Produkt sowie Mitteln (7) zum Ausstossen oder Ansaugen am vorderen Ende des Körpers und gegebenenfalls eine den genannten Körper umgebende halb-steife Umhüllung (9) aufweist, welche Hülle aus einem rohrförmigen dünnwandigen Element (1, 7, 9) umschliesst, wobei ein Ende (13c, 25, 25a, 25b, 25c, 25d) des genannten Elementes dicht aber zerreissbar verschlossen ist, wobei die Länge des genannten Elementes ungefähr jener der Pistolen-Einheit (1, 7, 9) entspricht, wobei weiters zwei annähernd parallele Schlitze (16) in der Nähe jenes Endes (13a) vorgesehen sind, das dem genannten verschlossenen Ende gegenüberliegt.

2. Schutzhülle nach Anspruch 1, dadurch gekennzeichnet, dass das rohrförmige Element (13, 24, 24a, 24b, 24c, 24d, 30) durch Extrudieren hergestellt und so ausgebildet ist, dass es durch Flachdrücken zwei übereinanderliegende Schichten bildet, wobei das verschlossene Ende dieser Hülle (13c, 25, 25a, 25b, 25c, 25d) durch eine Verbindung der genannten Schichten in Querrichtung zur Achse des rohrförmigen Elementes gebildet ist.

3. Schutzhülle nach Anspruch 1, dadurch gekennzeichnet, dass das rohrförmige Element flachgedrückt vorliegt und aus zwei entlang ihrer Ränder zusammengeschweissten Bahnen aus verschiedenen Materialien besteht.

4. Schutzhülle nach einem der Ansprüche 2 oder 3, dadurch gekennzeichnet, dass die beiden Schlitze (16) jeweils in den beiden übereinanderliegenden Schichten ausgeführt und übereinanderliegend vorgesehen sind.

5. Schutzhülle nach einem der Ansprüche 1 bis 4, dadurch gekennzeichnet, dass die beiden Schlitze (16) parallel zur Achse des rohrförmigen Elementes (13, 24, 24a, 24b, 24c, 24d, 30) ausgerichtet sind.

6. Schutzhülle nach einem der Ansprüche 1 bis 5, dadurch gekennzeichnet, dass einer der beiden Teile eines normalen Querschnittes des rohrförmigen Elementes, die durch eine Linie begrenzt sind, die die Schnittpunkte zwischen dem Querschnitt und den beiden Schlitzen (16) verbindet, wenigstens dem Querschnitt der Pistole entspricht.

7. Schutzhülle nach einem der Ansprüche 2 bis 6, wenn die Ansprüche 5 und 6 auf Anspruch 2 rückbezogen sind, dadurch gekennzeichnet, dass die beiden Schichten entlang einer geraden, zur Achse des rohrförmigen Elementes (24) rechtwinkeligen Linie (25) verbunden sind.

8. Schutzhülle nach einem der Ansprüche 2 bis 6, wenn die Ansprüche 5 und 6 auf Anspruch 2 rückbezogen sind, dadurch gekennzeichnet, dass die beiden Schichten entlang einer geraden Linie (26a) verbunden sind, die zur Achse des rohrförmigen Elementes (24a) annähernd gleich geneigt ist wie zur Transversalen.

9. Schutzhülle nach einem der Ansprüche 2 bis 6, wenn die Ansprüche 5 und 6 auf Anspruch 2 rückbezogen sind, dadurch gekennzeichnet, dass die beiden Schichten entlang einer geraden Linie (25b) verbunden sind, die sehr stark mit Bezug auf die Transversale geneigt ist.

10. Schutzhülle nach Anspruch 9, dadurch gekennzeichnet, dass die Linie (25c) an jedem ihrer beiden Enden abgerundet (26) oder durch eine kurze Querlinie unterbrochen ist.

11. Schutzhülle nach einem der Ansprüche 2 bis 6, wenn die Ansprüche 5 und 6 auf Anspruch 2 rückbezogen sind, dadurch gekennzeichnet, dass die beiden Schichten entlang einer quergerichteten halbkreisförmigen Linie (25d) verbunden sind.

12. Schutzhülle nach einem der Ansprüche 7 bis 11, dadurch gekennzeichnet, dass sie Teil eines extrudierten kontinuierlichen und flachgedrückten Schlauches (14) ist, der aus einer axialen Aneinanderreihung einer Reihe solcher Hüllen (13, 24, 24a, 24b, 24c, 24d) besteht.

13. Schutzhülle nach Anspruch 12, wenn er auf einen der Ansprüche 7 und 8 rückbezogen ist, dadurch gekennzeichnet, dass die Hüllen (13, 24, 24a) durch Anordnung im gleichen Sinn aneinandergereiht sind, wobei die Verbindungslinie (13b, 13c, 25, 25a) einer Hülle direkt an das offene Ende (13a) der benachbarten Hülle angrenzt.

14. Schutzhülle nach Anspruch 12, wenn er auf einen der Ansprüche 9 bis 11 rückbezogen ist, dadurch gekennzeichnet, dass die Hüllen (24b, 24c, 24d) so paarweise Kopf zu Ende aneinandergereiht sind, dass ihre Verbindungslinien (25b, 25c, 25d) einander benachbart sind.

15. Schutzhülle nach Anspruch 14, dadurch gekennzeichnet, dass die Verbindungslinien (25c) von zwei benachbarten Kopf zu Ende angeordneten Hüllen (24c) durch eine Schwächungslinie voneinander getrennt sind.

16. Schutzhülle nach einem der Ansprüche 2 bis 15, wenn die Ansprüche 5 bis 15 auf Anspruch 2 rückbezogen sind, dadurch gekennzeichnet, dass jede Verbindungslinie (13c, 25, 25a, 25b, 25c, 25d) eine Schweisslinie ist.

17. Schutzhülle nach einem der Ansprüche 12 bis 15 und 16, wenn Anspruch 16 auf einen der Ansprüche 12 bis 15 rückbezogen ist, dadurch gekennzeichnet, dass der extrudierte, kontinuierliche und flachgedrückte Schlauch (14), von dem ein Teil die Hülle ist, axial in Form einer Rolle aufgerollt ist, die in einem Spender (17) von im wesentlichen zylindrischer Form enthalten ist, der einen Schlitz (18) aufweist, durch den der genannte Schlauch austritt.

18. Schutzhülle nach Anspruch 17, dadurch gekennzeichnet, dass der den Schlauch, von dem sie ein Teil ist, enthaltende Spender (17) selbst abnehmbar auf einer festen Ausgabevorrichtung (20) montiert ist.

19. Schutzhülle nach Anspruch 17, dadurch gekennzeichnet, dass sie Teil einer Hüllenrolle (19) ist, die ihrerseits in Kettenform (29) vorliegt.

20. Schutzhülle nach einem der Ansprüche 7 bis 11, dadurch gekennzeichnet, dass sie einen Teil eines Stapels solcher Hüllen (30) bildet, die flachgedrückt und übereinanderliegend in die gleiche Richtung ausgerichtet sind, welcher Stapel durch eine Bindung (31) an seinem den Verbindungslinien gegenüberliegenden Ende (13a) gehalten ist.

FIG.1

FIG.2

FIG.3

FIG.4

0 093 630

FIG.5

25    24    14    16

FIG.6

25a    24a    14    16

FIG.7

16    24b    25b    24b 14

FIG.8

27    26  26    25c    24c    14
16    24c  25c 28    27  26  26

FIG.10

19    29

FIG.9

24d    25d    24d    14

FIG.11

31    13a    16    30

0 093 630